# EUROPEAN PATENT APPLICATION

(11) **EP 4 806 424 A1**
(43) Date of publication of application: **16.09.2026**
(21) Application number: 25163334.3
(22) Date of filing: 12.03.2025
(51) Int. Cl.: A61K 9/00, A61K 9/08, A61K 31/167, A61K 47/26

(54) **READY-TO-INJECT HYPERBARIC PRILOCAIN SOLUTION**

(71) Applicant: B. Braun Melsungen AG, 34212 Melsungen (DE)
(72) Inventor: FISCHER, Oliver, 34212 Melsungen (DE); KERSTE, Eric, 34212 Melsungen (DE)
(74) Representative: Maiwald GmbH

(57) **Abstract**

The present disclosure relates to a pharmaceutical formulation and packaging system for prilocaine injectable solutions containing glucose intended for intrathecal administration. Specifically, the disclosure provides an optimized packaging and preparation method that enhances the stability and safety of prilocaine solutions without the need for costly inert conditions. The preparation method of the present disclosure improves shelf-life and reduces the risk of degradation of prilocaine and glucose, by maintaining a stable pH value, surprisingly also under atmospheric conditions. The omission of inert conditions during production and storage simplifies handling and eliminating the safety risks associated with glass ampoules.

## Description

### Technical Field

The present disclosure relates to the field of pharmaceutical formulations, specifically to a ready to inject hyperbaric solution for injection, preferably intrathecal injection, containing prilocaine and glucose. More particularly, the disclosure concerns improved packaging and preparation methods for prilocaine solutions containing glucose specifically suitable for intrathecal administration.

### Background

Prilocaine is a widely used local anaesthetic agent known for its efficacy in producing nerve block and infiltration anaesthesia.

Intrathecal injections of prilocaine solutions have gained attention due to their rapid onset and efficacy in surgical, obstetric, and pain management applications. Traditionally, prilocaine solutions for intrathecal injections are packaged in glass ampoules, such as the commercially available Takipril^{®}. This packaging approach, however, presents several limitations, as well as handling challenges for healthcare providers.

One of the primary challenges with current prilocaine packaging lies in the use of glass ampoules, which - although commonly employed - are associated with significant disadvantages. Firstly, glass ampoules are susceptible to breakage and/or chipping of glass splinters when opening a glass ampoule, thereby creating a risk of contamination of the prilocaine formulation with glass particles. This can lead to severe health risks, potential patient injury and it may require additional steps, such as filtration of the formulation, before injection. Additionally, breakage of ampoules can cause injury to healthcare providers, increasing the risk of sharps-related incidents.

A further limitation of glass ampoules is that they cannot effectively protect prilocaine from environmental exposure after opening, which is particularly problematic if only a partial dose is required and administered. In many cases, healthcare professionals may need to dispose the remaining solution due to the risk of contamination or degradation. Consequently, the inability to reseal glass ampoules leads to higher waste and increased costs of therapy.

Moreover, hyperbaric prilocaine solutions are particularly sensitive to oxidation and (oxidative) degradation, that are usually addressed by using inert conditions during manufacturing and packaging into glass ampoules or the like. EP 1 988 924 A2 relates to the packaging of a hyperbaric solution of prilocaine under inert gas due to the instability under atmospheric conditions and oxygen. Such inert conditions, often achieved using nitrogen or argon gas, are costly and introduce manufacturing complexity. Inert packaging conditions require specialized facilities and equipment, which can increase the costs of the product and limit its accessibility, particularly in regions with limited pharmaceutical infrastructure. Additionally, re-sealing under inert conditions is not possible for healthcare providers.

Given these limitations, there is a growing need for improved packaging solutions for hyperbaric prilocaine solutions that provide enhanced protection without requiring inert conditions. Such solutions would improve the handling, safety and stability of prilocaine formulations, ensuring that the active ingredient remains effective over time and under typical storage conditions, that the prilocaine solution still has the required concentration (or amount) of prilocaine, and that side products are reduced. Developing new packaging and preparation options that can maintain the stability of prilocaine without the reliance on costly inert environments represents a significant advancement in the field. These improved solutions would make prilocaine more accessible, reduce waste, and mitigate safety risks for both patients and healthcare providers. Improving the packaging and preparation methods of prilocaine injection solutions is therefore advantageous to enhance both safety and stability.

### Summary

The inventors of the present disclosure surprisingly found that hyperbaric prilocaine solutions containing glucose are storage stable over a prolonged period if the pH of the solution is above a specific threshold at the beginning of the storage period. The storage stability of the solution may be monitored by measuring the concentration of an impurity of glucose, 5-hydroxymethylfurfural ("HMF"). By providing a hyperbaric prilocaine solution with a pH above 5.5 at the beginning of the storage period, the above mentioned problems may be successfully addressed. In particular, the hyperbaric prilocaine solution of the present disclosure may be packaged without the need of inert conditions and the use of glass packaging. Glass packaging is used for its ability to be impermeable for gas, which is not the case in polymer based packaging. This results in a process that allows for cost effective packaging and storing, as well as efficient handling during and after use.

In a first aspect, the present disclosure relates to a pharmaceutical hyperbaric stable aqueous solution for injection comprising prilocaine, or a pharmaceutically acceptable salt thereof, and glucose, wherein the solution has a 5-hydroxymethylfurfural concentration of less than 24 µg/mL over a storage period of at least five years; and wherein the solution has a pH ≥ 5.5 at the beginning of the storage period.

In a second aspect, the present disclosure relates to packaging of a solution according to the first aspect wherein the packaging is oxygen permeable and/or allows for atmospheric conditions inside the packaging.

In a third aspect, the present disclosure relates to a method for preparing the hyperbaric stable solution of the present disclosure, comprising the following steps in the order of:
(a) Dissolving prilocaine or a pharmaceutical acceptable salt thereof and glucose in water for injection, thereby providing an aqueous solution;
(b) Adjusting the pH value of the aqueous solution to ≥ 5.5 by addition of a suitable base or a suitable acid, thereby providing a pH-adjusted aqueous solution;
(c) Optionally sterilizing the pH-adjusted aqueous solution by filtration;
(d) Distributing the pH-adjusted aqueous solution into a packaging, resulting in a filled packaging; and
(e) Optionally heat sterilizing the filled packaging.

The inventors of the present disclosure surprisingly and unexpectedly found that a pH value in a specific range is enhancing the stability during the storage period of the injectable prilocaine solution to prevent or reduce the formation of 5-hydroxymethylfurfural and maintain its amount (%-HMF) equal to or less than a limit of 24 µg/mL, and further reduce other decomposition products throughout the entire storage period.

With this surprising and unexpected finding, the hyperbaric prilocaine solution of the first aspect may be produced and distributed into a packaging in a procedure not requiring inert conditions, and which further allows for a storage under atmospheric conditions. The injectable prilocaine solutions can be advantageously stored in an oxygen or air permeable packaging, or any other kind of packaging, thereby also avoiding the risks and disadvantages associated with commonly used glass ampoules.

### Brief Description of the Drawings

- Fig. 1:: pH value development at different starting pH values during storage at 40 °C.
- Fig. 2:: 5-HMF development at different starting pH values during storage at 40 °C.
- Fig. 3:: 2-methylaniline development during storage at 40 °C.
- Fig. 4:: Influence of oxygen on the pH value.
- Fig. 5:: Prilocaine concentration at different starting pH values after 6 months storage at 40 °C.

### Detailed Description of the disclosure

The different aspects of the present disclosure are described in the following in more detail, exemplified by preferred embodiments and embodiment examples. However, it is understood that the scope of the present disclosure is not limited thereto, but only by the appendant claims.

The present disclosure relates to three aspects. These three aspects are explained in more detail in the following by explaining and providing preferred embodiments to these aspects.

In a first aspect, the present disclosure relates to a pharmaceutical hyperbaric stable aqueous solution for injection comprising prilocaine, or a pharmaceutically acceptable salt thereof, and glucose, wherein the solution has a 5-hydroxymethylfurfural concentration of less than 24 µg/mL over a storage period of at least five years; and wherein the solution has a pH ≥ 5.5 at the beginning of the storage period.

### The pharmaceutical hyperbaric stable aqueous solution for injection

According to a first aspect, the present disclosure relates to a pharmaceutical hyperbaric stable aqueous solution for injection comprising prilocaine, or a pharmaceutically acceptable salt thereof, and glucose, wherein the solution has a 5-hydroxymethylfurfural concentration of less than 24 µg/mL over a storage period of at least five years; and wherein the solution has a pH ≥ 5.5 at the beginning of the storage period.

The solution of the present disclosure is a hyperbaric prilocaine aqueous solution. The solution of the present disclosure thus contains at least prilocaine dissolved in an aqueous injectable medium. The prilocaine may be used as prilocaine free base, or as a pharmaceutically acceptable salt of prilocaine. In the following, when reference is made to prilocaine, i.e., prilocaine in general and not specifically as free base, this is understood to comprise prilocaine free base or a pharmaceutically acceptable salt thereof. Similarly, when referring to a prilocaine solution, this refers to a solution of prilocaine free base or a pharmaceutically acceptable salt of prilocaine.

In a preferred embodiment, the aqueous injectable medium is water for injection. In another preferred embodiment, the aqueous injectable medium may be deionized water.

The hyperbaric solution of the present disclosure has a density higher than that of the spinal fluid of a vertebrate, preferably of a mammal, further preferably of a human. In the present disclosure, the increase in density is achieved by adding glucose to the prilocaine aqueous solution. Accordingly, the pharmaceutical hyperbaric stable aqueous solution of the present disclosure contains glucose, thereby rendering the prilocaine solution to a hyperbaric prilocaine solution.

The pharmaceutical hyperbaric stable aqueous solution for injection of the present disclosure thus at least contains prilocaine or a pharmaceutically acceptable salt thereof, glucose and water.

In a preferred embodiment, the pharmaceutical hyperbaric stable aqueous solution for injection of the present disclosure consists of prilocaine or a pharmaceutically acceptable salt thereof, glucose and water. It is understood that this refers to the ingredients added to result in the solution, and does not comprise any impurities which may be present in the solution when prepared, or which may develop during storage of the solution.

### Prilocaine

In a preferred embodiment, the solution of the present disclosure comprises prilocaine as the free base.

In another preferred embodiment, the solution of the present disclosure comprises prilocaine as a pharmaceutically acceptable salt. It is particularly preferred to use prilocaine hydrochloride, i.e., prilocaine HCl.

The concentration of prilocaine in the hyperbaric solution can vary depending on the formulation and the intended clinical use. Usually 1 to 5 % (w/w) is standard in most hyperbaric prilocaine products. The amount of prilocaine in % (w/w) in the hyperbaric solution of the present disclosure is calculated on the basis of the amount (weight) of prilocaine hydrochloride relative to the total amount (weight) of the final solution.

In a preferred embodiment, the solution of the present disclosure has an amount of prilocaine or a pharmaceutically acceptable salt thereof between 1.5 and 2.5 % (w/w), preferably between 1.8 and 2.2 % (w/w), more preferably between 1.9 and 2.1% (w/w) and most preferably at about 2 % (w/w).

The amounts of prilocaine base or a pharmaceutically acceptable salt other than hydrochloride have to be adjusted in accordance to the values determined for prilocaine hydrochloride, when preparing a solution according to the present disclosure using prilocaine base or a pharmaceutically acceptable salt other than hydrochloride.

During storage of the hyperbaric prilocaine solution, the solution may decompose. In other words, the components of the solution may decompose, thereby resulting in decomposition products. The main decomposition product of prilocaine is 2-methylaniline. The decomposition of prilocaine is disadvantageous for two reasons. At first, the amount of active agent, i.e., prilocaine, is reduced, thereby reducing the efficacy of the analgesic. Secondly, decomposition products are side products which may be harmful, and these decomposition products may lead to undesired side effects. For these reasons, limits for side products or decomposition products are regularly defined. The limit for 2-methylaniline according to the specification of the Pharmacopoeia Europaea monography 1363 (Ph. Eur. 11. 4) for Prilocaine Hydrochloride or Pharmacopoeia Europaea monography 1362 (Ph. Eur. 11. 4) for Prilocaine free base is at a concentration of 100 ppm 2-methylaniline. Derived from this, a limit of 0.1% of the area of the HPLC peak for 2-methylaniline relative to the area of the HPLC peak for prilocaine was defined. The solution according to the present disclosure is within this limit for at least 15 months of storage at 40 °C, corresponding to a storage period of 5 years at a temperature of 25 °C.

In a preferred embodiment, the solution of the present disclosure has an amount of less than 0.03 % of 2-methylaniline, relative to the prilocaine concentration after at least 15 months of accelerated storage at 40 °C, corresponding to a storage of 5 years at a temperature of 25 °C.

In another preferred embodiment, the solution of the present disclosure has an amount of less than 0.05 % of 2-methylaniline relative to the prilocaine concentration after at least six months of storage at 40 °C, corresponding to a storage of 2 years at a temperature of 25 °C.

### Glucose

In hyperbaric solutions, glucose serves as a densifying agent to increase the solution's specific density, making it denser than cerebrospinal fluid. This hyperbaric property allows for the solution to be precisely positioned within the subarachnoid space during spinal anaesthesia, enabling targeted and controlled distribution of the anaesthetic agent. Glucose is used to achieve the desired osmolarity and ensuring predictable clinical outcomes.

In a preferred embodiment, the solution of the present disclosure has an osmolarity in the range of from 500 to 560 mOsm/kg, preferably in a range of from 520 to 550 mOsm/kg, and more preferably in a range of from 525 to 540 mOsm/kg.

In a particularly preferred embodiment, the solution of the present disclosure has an osmolarity in the range of from 530 to 535 mOsm/kg.

An osmolarity in this range allows for the prilocaine solution to be injected into, e.g., a spinal cord of a vertebrate, preferably of a mammal, further preferably of a human.

The solutions of the present disclosure may contain any form of glucose that is commonly used for injectable solutions. The solution may contain glucose monohydrate, anhydrous glucose or dextrose, and preferably the solution contains glucose monohydrate.

In a preferred embodiment, the amount of glucose in the solution is between 5 and 7 %(w/w), preferably between 5.4 and 6.6 %(w/w), more preferably between 5.6 and 6.4 %(w/w), even more preferably between 5.8 and 6.2 %(w/w) and most preferably at about 6 % (w/w), based on the total amount of the final solution. The amounts described herein refer to anhydrous glucose. By using these specific amounts of glucose, the prilocaine solution has a osmolarity in the desired range, rendering the solution to a hyperbaric solution. When different amounts of glucose are used, the osmolarity of the solution may be adjusted to the needs.

### 5-Hydroxymethylfurfuraldehyde as impurity

In a pharmaceutical formulation of hyperbaric glucose-containing prilocaine solutions, the presence of 5-hydroxymethylfurfuraldehyde (5-HMF) as an impurity is a concern due to its potential impact on product safety and efficacy. It is assumed that 5-HMF is a degradation product formed from glucose. In the context of the hyperbaric glucose-containing solutions of the present disclosure, this impurity can arise during manufacturing or storage.

5-HMF may be formed from glucose under acidic conditions through a series of dehydration reactions. In an acidic environment, the protonation of glucose enhances its reactivity, facilitating the conversion of the glucose into its enol form. This intermediate undergoes further transformations, including dehydration, to form 5-HMF. A low pH acts as a catalyst because the high concentration of protons accelerates the breakdown of glucose and stabilizes reaction intermediates, leading to an efficient conversion process.

The chemical reactivity of 5-HMF poses a dual risk in such solutions. At first, it may interact with the active pharmaceutical ingredient, prilocaine, potentially compromising its stability or therapeutic efficacy. Secondly, 5-HMF is recognized as a potential toxicant, with evidence linking its accumulation to cytotoxicity and adverse biological effects. Due to these considerations, control measures to limit its formation and to ensure patient safety are recommended.

In a preferred embodiment, the solution of the present disclosure has a 5-hydroxymethylfurfural content of less than 20 µg/mL, preferably less than 15 µg/mL, further preferably 10 µg/mL, and still further preferably less than 7 µg/mL over the entire storage period. The entire storage period is thereby understood to be at least five years under normal storage conditions assuming standard temperature of 25 °C.

In a further preferred embodiment, the solution of the present disclosure has a 5-hydroxymethylfurfural content of less than 7 µg/mL, preferably less than 6 µg/mL, further preferably less than 5 µg/mL, and still further preferably less than 4 µg/mL after storage for 6 months at 40 °C, and/or the solution has a 5-hydroxymethylfurfural content of less than 4 µg/mL, preferably less than 3 µg/mL, further preferably less than 2 µg/mL after storage for 2 months at 40 °C.

A six-months storage period under accelerated conditions at 40 °C corresponds to a storage period of 2 years at a temperature of 25 °C.

### Sterilization

Sterilization of injectable solutions is essential to eliminate microbial contamination, ensuring patient safety and compliance with regulatory standards. Injectable products must be sterile to prevent infections and other complications during administration. Common sterilization methods for injectable solutions include sterile filtration, autoclaving, radiation or aseptic processing. The choice of sterilization method depends on the solution's composition, stability, and packaging material, ensuring effective sterilization while maintaining product integrity and safety.

The solution with an adjusted pH according to the present disclosure can surprisingly be handled and stored under standard atmospheric conditions without the need for controlled environments, aseptic and inert processing during the whole preparation, manufacture and packaging is thus not required.

However, aseptic processing and/or the use of inert conditions is explicitly not excluded in the methods of the present disclosure.

In a preferred embodiment, the solution of the present disclosure is sterilized by heat sterilization. In such preferred embodiment, the sterilization may be achieved at a temperature of at least 110 °C, preferably at least 121 °C. Independent of the temperature, the sterilizing may be performed with a minimum lethality of F₀ ≥ 8 minutes, preferably F₀ ≥ 15 minutes.

In a further preferred embodiment, the solution of the present disclosure is sterilized at a temperature of at least 110 °C with an F₀ value ≥ 8 minutes.

In another preferred embodiment, the solution of the present disclosure is sterilized at a temperature of at least 121 °C with an F₀ value ≥ 15 minutes.

As used herein, when heat sterilization is performed for a specific period, said period refers to the F₀ value. F₀ is a quantitative parameter used to evaluate the amount of heat transferred to a product or solution during a heat sterilization process, and describes the lethality performance of the process.

According to one embodiment, F₀ can be calculated through the following formula: ${F}_{0} = {\int }_{0}^{t} {10}^{\frac{T - 121 ° C}{Z}} dt$ wherein Z is 10 °C, and T is the temperature of the container at time point t. This formula can be applied when the temperature T of the container changes during sterilization.

In practice, the sterilizer, such as an autoclave or water cascade sterilizer, heats the product (e.g., container) for a certain period of time until the sterilization temperature of, e.g., 121 °C is reached. During the heating time for heating from 100 °C to 121 °C, and also during the cooling time, microorganisms are killed. Taking this killing effect during the heating and cooling time into account, the holding time can be shortened accordingly. Thus, the desired F₀ value can be achieved with a shorter overall process time.

In other words, the F₀ value of a terminal sterilization process is a measure for the lethality, expressed in terms of the equivalent time in minutes at a temperature T of 121 °C delivered by the process to the load in its container with reference to micro-organisms possessing a theoretical Z-value of 10 °C. For instance, an F₀ value of 1 min is the heat effect (lethal effect) of 121 °C within one minute.

According to another embodiment, the period F₀ for which sterilization is performed, is calculated on basis of the time t for which the container is held at a constant temperature T. If the container is held at a constant temperature T for a total time t during sterilization, the above formula simplifies as follows: ${F}_{0} = t ⋅ {10}^{\frac{T - 121 ° C}{Z}}$ wherein Z is 10 °C, T is the temperature of the container, and t is the time for which the container is held at temperature T.

Furthermore, if the container is held at a temperature T of 110 °C for 101 min, the above equation simplifies to ${F}_{0} = 101 min ⋅ {10}^{\frac{110 ° C - 121 ° C}{10 ° C}} = 101 min ⋅ {10}^{- 1.1} \approx 8.0 min$

In other words, a 101 min sterilization at 110 °C is equivalent, in terms of lethal effect, to about 8.0 min at 121 °C.

In an alternative preferred embodiment, the solution of the present disclosure is sterilized by filtration sterilization, combined with aseptic processing of the solution.

The inventors of the present disclosure surprisingly found that a solution having a pH of at least 5.5 at the beginning of the storage period allows for a storage period of at least 5 years. It is understood that the packaged solution is in a sterile condition at the beginning of the storage period, and also throughout the entire storage period. It was outlined that sterilization may be achieved by different means; the two mostly used methods are heat sterilization and sterile filtration. While heat sterilization does not require aseptic handling during packaging and distribution of the solution, sterile filtration requires the use of aseptic conditions and also the use of sterile packaging materials.

The way of sterilization, however, has an influence on the pH of the solution. While the pH of the solution is not or not substantially changed during sterile filtration, the use of heat sterilization may change the pH of the packaged solution. It was found that the pH is usually reduced during heat sterilization. Without being bound by theory, it is assumed that the harsher conditions of heat sterilization, i.e., the high temperature over a prolonged period of time, accelerate the partial degradation of the components of the solution, thereby lowering the pH of the solution.

It was experimentally found that the reduction in pH during heat sterilization appears to at least depend on the pH prior to heat sterilization, as outlined in Example 2 and Table 1. From these Examples, it may be concluded that a lower pH is decreased by a higher value than a relatively higher pH. In other words, the lower the pH prior to heat sterilization, the higher the relative reduction of pH during heat sterilization, if the pH prior to heat sterilization is in the range of from 5.0 to 6.5.

For the solution of the present disclosure, the pH after sterilization and at the beginning of the storage period is to be considered. In other words, the pH of the solution at the beginning of the storage period, which is after sterilization, is equal to or above 5.5 ( ≥ 5.5). This pH value has to be met irrespective of the sterilization method.

### pH value

As outlined above, the degradation of prilocaine and/or glucose may lead to the generation of impurities, in particular 2-methylaniline and 5-HMF. Regulatory guidelines typically require the quantification and specification of such impurities in drug formulations. Analytical techniques, such as high-performance liquid chromatography (HPLC), are employed to detect and measure 5-HMF at trace levels. It was surprisingly found by the inventors of the present disclosure that a formulation strategy including the optimization of the pH in the solution can mitigate the generation of 5-HMF during the product's lifecycle.

It was found that the formation of 5-HMF is significantly accelerated in hyperbaric prilocaine solutions at lower pH values. Without being bound by theory, it is assumed that acidic conditions catalyse the dehydration of hexoses, such as glucose, leading to an increase in 5-HMF. The inventors further found that, to minimize the generation of 5-HMF impurity, a careful pH control is required. It is preferred to control the pH value at the beginning of the storage period, i.e., after packaging and sterilization.

It was surprisingly found that a low amount of impurities, in particular 5-HMF at a concentration of less than 24 µg/mL, over a storage period of at least five years may be achieved when the hyperbaric glucose-containing prilocaine solution has a pH value of equal to or above 5.5 ( ≥ 5.5) at the beginning of the storage period.

In a preferred embodiment, the solution as a pH of ≥ 5.7, preferably ≥ 5.9, more preferably ≥ 6.0 or > 6.0, even more preferably ≥ 6.1, and most preferably ≥ 6.2, at the beginning of the storage period.

For a solution to have a specific pH at the beginning of the storage period, i.e., after sterilization, it may be necessary to further increase the pH of the solution prior to sterilization if heat sterilization is used. Accordingly, in a preferred embodiment, the pH value is adjusted to ≥ 5.8, preferably adjusted to ≥ 6.0, still further preferably adjusted to ≥ 6.5 prior to a heat sterilizing step. This allows for a solution to be obtained having a pH in the required range after sterilization by heat.

### Use in intrathecal injections

In a preferred embodiment, the present disclosure relates to a solution according to the first aspect for use in intrathecal injections, preferably for anaesthesia.

Prilocaine is a local anesthetic commonly used in regional anesthesia, including intrathecal (spinal) injections. In intrathecal anesthesia, prilocaine is administered into the cerebrospinal fluid to block nerve transmission, providing rapid and effective anesthesia for lower body procedures such as urological, gynecological, or orthopedic surgeries. Its favorable pharmacokinetic profile, including rapid onset and moderate duration of action, makes it suitable for short to intermediate procedures. Furthermore, prilocaine is associated with a lower incidence of transient neurologic symptoms compared to some alternatives, like lidocaine.

Hyperbaric prilocaine solutions, typically combined with glucose, are used to control the drug's distribution in the subarachnoid space, allowing precise targeting of the anesthetic effect. Prilocaine's low risk of systemic toxicity and minimal impact on cardiovascular and respiratory systems further enhance its suitability for spinal anesthesia.

### The packaging

According to a second aspect, the present disclosure relates to packaging of a solution according to the first aspect wherein the packaging is oxygen permeable and/or allows for atmospheric conditions inside the packaging.

Prilocaine injectables are often manufactured under an inert atmosphere, such as nitrogen, in the understanding to prevent the degradation of the drug and/or excipients, and to preserve its stability. Prilocaine, like many local anesthetics, is understood to be susceptible to oxidation, which may lead to the formation of undesirable metabolites or impurities. By creating an inert atmosphere during manufacturing and/or storage, the presence of oxygen is minimized, thereby reducing the risk of oxidation and ensuring the quality and safety of the final product and during shelf-life.

To maintain the exclusion of atmospheric conditions, in particular oxygen, the solution has to be filled and stored in a packaging that is impermeable to ingress of air and/or oxygen and able to be hermetically sealed. Glass vials are commonly used to store oxygen-sensitive solutions due to their hermetic sealing.

The solution according to the present disclosure was surprisingly found storage-stable when manufactured and/or stored without the exclusion of oxygen or the exclusion of atmospheric conditions.

Packaging that does not require inert conditions offers several significant advantages in both the packaging design and the filling process.

The need for specialized equipment and the use of inert gases like nitrogen, which are typically required to prevent oxidation and preserve product stability, are eliminated. This reduces production costs and simplifies storage and transport, as the packaging can be handled and stored under standard atmospheric conditions without the need for controlled environments. Additionally, such packaging can contribute to a more sustainable manufacturing process, as it avoids the use of energy-intensive inert gases and reduces waste associated with maintaining a controlled atmosphere.

In terms of the filling process, the absence of a need for inert conditions increases operational efficiency. The filling process can be streamlined, as there is no requirement to create or maintain specialized conditions, which can speed up production timelines. This also reduces the risk of contamination during filling since traditional methods often involve complex systems designed to avoid exposure to oxygen. With packaging that is less sensitive to atmospheric conditions, the process becomes simpler and less prone to handling issues. Overall, packaging that doesn't rely on inert conditions enhances both the efficiency and flexibility of the manufacturing process, leading to a more straightforward, cost-effective, and environmentally friendly production cycle. Also the use of materials that are not air-tight adds to this advantage.

In general, when a solution is packaged into a vial, there is a headspace (gas) in the vial in addition to the solution (liquid). Said headspace may have to be filled with an inert gas if inert handling is required. Such headspace may have an amount of below 20 % (v/v), such as below 10 % (v/v) or below 5 % (v/v), based on the volume of the liquid in the packaging.

In a preferred embodiment, the packaging according to the present disclosure contains a headspace, and the headspace contains more than 2 % of oxygen, preferably more than 5 % of oxygen, more preferably more than 10°% of oxygen and most preferably more than 20 % of oxygen.

The packaging of a liquid injectable solution may comprise a glass vial or a vial made of a polymeric material. The glass vial may be closed with a lid, such as a lid made of a polymeric material, or it may be a sealed glass ampoule, being made entirely of glass. The vial made of a polymeric material may also be sealed by a lid, such as a lid made of a polymeric material, or it may be sealed ampoule, being made entirely of a polymeric material.

In a preferred embodiment, the packaging according to the present disclosure comprises at least one polymer, wherein the polymer is selected from the group consisting of a polyethylene, a polypropylene, a polyvinylchloride, a polystyle, a polyamide, a polylactic acid, a polycarbonate, a polyacrylonitrile, a polyester, a cyclic olefin copolymer (COCs), a cyclic olefin polymer (COPs) and combinations thereof. In other words, the packaging of the liquid injectable solution of the present disclosure comprises at last a part being made of a polymeric material, i.e., comprising at least one polymer, or it may entirely be made of a polymeric material, i.e., comprising at least one polymer. Such packaging is referred to a polymer packaging in the present disclosure.

Polymer packaging offers several advantages over traditional glass vials, especially glass ampoules, particularly in the context of injectable solutions. One key benefit is its lightweight nature, which reduces transportation costs and handling risks compared to glass vials, which are heavier and more prone to breakage.

The reduced risk of breakage leads to fewer product losses during handling and distribution, improving overall safety. Polymer packaging is also more cost-effective, particularly when considering mass production and the elimination of the need for sterilization processes required for glass. These advantages make polymer packaging a superior choice for injectable solutions, offering practical, economic, and performance-related benefits over traditional glass containers.

Polymer packaging also offers greater flexibility in design, allowing for more customizable shapes, sizes, and sealing options that can enhance product stability and ease of use.

The packing according to the present disclosure is in particular not limited to the polymers listed herein. The solution according to the present disclosure may be stored in a packing of any material that is suitable therefore, including all common packaging materials of any shape and size.

Preferably, the packaging should withstand high temperatures required during processes like heat sterilization, typically around 121 °C. Materials used in such packaging are selected for their ability to maintain structural integrity, prevent degradation, and preserve the sterility of the product throughout the sterilization process. Common materials include certain types of medical-grade plastics, such as polypropylene, which offer good thermal resistance and can endure repeated exposure to high temperatures without warping or leaching harmful substances. Additionally, polymer-based films and laminated materials are often used, as they can be heat-sealed to create a sterile barrier while remaining resistant to thermal damage. Packaging systems designed for heat sterilization also typically include features like venting systems to allow for the safe release of steam, ensuring proper sterilization and preventing the buildup of pressure.

In a preferred embodiment, the packaging is sterilized by heat sterilization, at a temperature of at least 110 °C, preferably 121 °C. It is further preferred that the sterilizing is performed with an F₀ value ≥ 8 minutes, preferably with an F₀ value ≥ 15 minutes.

In a further preferred embodiment, the packaging of the present disclosure is sterilized at a temperature of at least 110 °C with an F₀ value ≥ 8 minutes.

In a more preferred embodiment, the packaging of the present disclosure is sterilized at a temperature of at least 121 °C with an F₀ value ≥ 15 minutes.

In another preferred embodiment, the solution of the present disclosure is sterilized by filtration sterilization before distribution into a packaging according to the present disclosure.

### Method of preparation

According to a third aspect, the present disclosure relates to a method for preparing the hyperbaric stable solution of the present disclosure, comprising the following steps in the order of:
(a) Dissolving prilocaine or a pharmaceutical acceptable salt thereof and glucose in water for injection, thereby providing an aqueous solution;
(b) Adjusting the pH value of the aqueous solution to ≥ 5.5 by addition of a suitable base or a suitable acid, thereby providing a pH-adjusted aqueous solution;
(c) Optionally sterilizing the pH-adjusted aqueous solution by filtration;
(d) Distributing the pH-adjusted aqueous solution into a packaging, resulting in a filled packaging; and
(e) Optionally heat sterilizing the filled packaging.

The inventors of the present disclosure surprisingly and unexpectedly found that the adjustment of the pH value ≥ 5.5 is helping for the stability during the storage period of the injectable prilocaine solution to prevent the formation of 5-hydroxymethylfurfural impurity and maintain its amount (%-HMF) equal to or less than 24 µg/mL and reduce other decomposition products throughout the entire storage period.

The method of the present disclosure surprisingly and unexpectedly also allows for non-inert preparation procedure during manufacturing and enables a storage under atmospheric conditions. The injectable prilocaine solutions can advantageously be stored in oxygen or air permeable packaging or any other kind of packaging, avoiding the risks and disadvantages associated with commonly used glass ampoules.

In a preferred embodiment according to the present disclosure, at least one of the steps (a) to (e) is performed under atmospheric conditions or under inert conditions, preferably all of steps (a) to (e) are performed under atmospheric conditions or under inert conditions.

In a particularly preferred embodiment, all steps (a) to (e), optionally excluding (c) and/or (e), are performed under atmospheric conditions.

Non-inert preparation under standard atmospheric conditions of injectable solutions offers several advantages, particularly in terms of simplicity, cost-effectiveness, and flexibility during manufacturing. By eliminating the need for an inert atmosphere, such as nitrogen or argon, manufacturers can reduce both the complexity and cost of the production process, as specialized equipment and gas supply systems are not required. This can lead to shorter production timelines and lower overall costs. Additionally, non-inert preparations are easier to handle and store, as they do not require controlled environments, making them more practical for large-scale distribution.

Non-inert conditions can also allow for greater versatility in packaging choices and the incorporation of active ingredients without concerns about oxygen exposure. These benefits make non-inert preparations a more accessible and efficient option for producing injectable solutions, while still maintaining product integrity and safety.

### Acid and base

In a preferred embodiment of the method of the present disclosure, the base as used in step (b) for adjusting the pH of the solution is selected from the group consisting of NaOH, KOH, Ca(OH)₂, Ca(OH)₂, NaHCO₃, Na₂CO₃, Na₃PO₄, CaCO₃, K₂CO₃, and KHCO₃.

A suitable base is usually used to adjust the pH within the specific range of the present disclosure, when the solution comprises prilocaine HCl or another pharmaceutically acceptable salt resulting in a pH value that lies below the specific pH range required by the method of the present disclosure.

In particular, the present disclosure is not limited to the bases listed herein. Any base that is suitable to adjust the pH in an injectable solution may be optionally used, in particular any pharmaceutically acceptable base.

A suitable base is usually used to adjust the pH within the specific range of the method of the present disclosure, when the solution comprises prilocaine HCl.

In a preferred embodiment of the method of the present disclosure, the acid as used in step (b) for adjusting the pH of the solution is selected from the group consisting of HCl, citric acid, H₃PO₄, acetic acid and lactic acid.

A suitable acid is usually used to adjust the pH within the specific range of the method of the present disclosure, when the solution comprises prilocaine as the free base.

In particular, present disclosure is not limited to the acids listed herein. Any acid that is suitable to adjust the pH in an injectable solution may be optionally used, in particular any pharmaceutically acceptable acid.

In a preferred embodiment of the method of the present disclosure, step (a) includes the addition of a suitable base or a suitable acid in helping the prilocaine base or salt thereof to be dissolved in water. The suitable acid or base added in step (a) may be the same or a different acid or base used in step (b) to adjust the pH of the solution. Any acid or base suitable to be used in step (b) may also be used in step (a).

### pH of solution

In a preferred embodiment of the method of the present disclosure, the pH is in a range of between 5.5 to 6.5 after step (d), or optionally after step (e), if present.

In a further preferred embodiment according to the method of the present disclosure, the pH is in a range of between 6 to 6.5 after step (d), or optionally after step (e), if present.

In a particular preferred embodiment according to the method of the present disclosure, the pH is 6.0, preferably 5.9, more preferably 5.8, even more preferably 5.7, and most preferably 5.5 after step (d), or optionally after step (e), if present.

In a preferred embodiment of the method of the present disclosure, the pH value in step (b) is adjusted ≥ 6 before sterilization, preferably when the sterilization is a heat sterilization. It was found that the pH value of the final solution is lowered during heat sterilization. The pH value remains the most stable when chosen equal or above 6 prior to heat sterilization.

In a particular preferred embodiment of the method of the present disclosure, the pH value in step (b) is adjusted in a range between 6 and 6.5, preferably 6.3, more preferably 6.2, even more preferably 6.1, and most preferably 6.0 before sterilization, preferably when the sterilization is a heat sterilization.

In another preferred embodiment of the method of the present disclosure, one of step (c) or step (e) is present, however, not both.

Step (e) refers to a terminal sterilization method, after the packing is filled and sealed. The sterilization method in step (e) may be any suitable sterilization method, preferably the sterilization method may be heat sterilization.

In a preferred embodiment according to the method of the present disclosure, the sterilizing is performed at a temperature of at least 110 °C, preferably 121 °C. It is further preferred that the sterilizing is performed with an F₀ value ≥ 8 minutes, preferably with an F₀ value ≥ 15 minutes.

In a further preferred embodiment of the method of the present disclosure, the sterilizing is performed at a temperature of at least 110 °C with an F₀ value ≥ 8 minutes.

In a particularly preferred embodiment according to the method of the present disclosure, the sterilizing is performed at a temperature of at least 121 °C with an F₀ value ≥ 15 minutes .

Sterilization of packaging filled with an injectable solution must ensure both the sterility of the product and the integrity of the packaging. Suitable methods include autoclaving or heat sterilization. These methods are ideal for solutions and also packaging materials that can withstand high temperatures, such as glass vials or certain medical-grade polymers like polypropylene.

Heat sterilization offers several advantages, making it a preferred method for sterilizing many pharmaceutical products, including injectable solutions and their packaging. It is highly effective at eliminating a wide range of microorganisms, including bacteria, viruses, and spores, ensuring robust sterility. The process is well-established, easily validated, and does not require the use of chemicals, which reduces the risk of residual contaminants in the final product. Heat sterilization is cost-efficient due to its simplicity and reliance on readily available steam or dry heat. Additionally, it is environmentally friendly compared to chemical methods. For heat-stable materials, heat sterilization ensures consistent and reliable sterility without compromising the integrity of the product or packaging.

For extremely heat-sensitive solutions, aseptic processing may be used, where the packaging and solution are sterilized separately, and filling is done in a sterile environment to avoid microbial contamination. Another common method is radiation sterilization, which employs gamma rays or electron beams to sterilize pre-filled containers without exposing them to heat. The choice of method depends on the solution's thermal sensitivity, packaging material, and regulatory requirements, ensuring both sterility and product stability.

Step (c) according to the method of the present disclosure, refers to a sterilization of the solution by filtration. The subsequent distribution step (d) according to the method of the present disclosure is then performed under sterile conditions into pre-sterilized packaging. The sterilization of the packaging prior to distribution according to the method of the present disclosure can be performed by any sterilization method that is suitable. Sterilization of the packaging prior to distribution and filling of the packaging is optional, but in particular performed when step (c) according to the method of the present disclosure is present.

In a preferred embodiment, steps (c) and (d) according to the method of the present disclosure, may be performed under aseptic conditions.

The filled packaging is sealed or closed using standard methods, depending on the packaging material. Sealing can optionally be hermetically sealed.

In a further aspect, the solution according to the present disclosure is prepared according to the method described herein.

### Definitions

In the following, definitions of terms frequently used herein is given as they are used and understood in the context of the present disclosure.

The term "hyperbaric" is used herein in the context of a hyperbaric solution used in spinal anaesthesia. Such hyperbaric solutions have a density greater than that of spinal fluid of a vertebrate, preferably of a mammal, further preferably of a human, allowing for the injection of these solution into the spinal fluid for local anaesthesia.

Where the term "comprising" is used in the present description and claims, it does not exclude other elements. For the purposes of the present invention, the term "consisting of" is considered to be a preferred embodiment of the term "comprising". If hereinafter a group is defined to comprise at least a certain number of elements, this is also to be understood to disclose a group which preferably consists only of these elements.

Where an indefinite or definite article is used when referring to a singular noun, e.g. "a", "an" or "the", this includes a plural of that noun unless specifically stated otherwise.

The term "at least one" refers to a number of 1 or more representations of the following term, in particular a polymer. The representations of the respective material, if present more than once, i.e., 2, 3, 4, or more times, may be independently selected from each other within the definition of the respective material. In particular, at least one refers to 1, 2, 3, 4 or 5 independently selected representations of the respective material, particularly preferred 1 or 2 times.

Terms like "obtainable" or "definable" and "obtained" or "defined" are used interchangeably. This, e.g., means that, unless the context clearly dictates otherwise, the term "obtained" does not mean to indicate that e.g. an embodiment must be obtained by e.g. the sequence of steps following the term "obtained" even though such a limited understanding is always included by the terms "obtained" or "defined" as a preferred embodiment.

When it is referred to the term "the packaging is permeable", it may be a material of the whole packaging and/or only parts of the packaging, such as a closure cap, that enables an ingress of air, including in particular oxygen, to the inside of the packaging. The packaging is understood to be closed and sealed by any type of closure including sealing, and it is in particular liquid tight, but not necessarily air tight. The material of the packaging is preferably selected from an oxygen and/or air permeable plastic made from one or more polymer(s), but not limited thereto.

The term "atmospheric conditions" refers to temperature and pressure, as well as air under normal atmospheric conditions. In the context of the present disclosure, normal temperature refers to a temperature of 25 °C, normal pressure refers to 1 bar (10⁵ Pa) and normal composition of air, including about 21 % of oxygen. Packaging or storing under atmospheric conditions thus refers to a packaging or storing under these conditions. When storing under atmospheric conditions, the humidity is maintained in a range of from 20 to 80 % relative humidity.

The term "storage period" refers to a specific period where the pharmaceutical hyperbaric stable aqueous solution for injection of the present disclosure is stored in a packaging. The packaging is stored under atmospheric conditions for the specified period, usually at least two years, preferably five years. Under normal circumstances, the storage period begins after packaging of the solution, including sealing the packaging, and optionally (heat) sterilizing the solution in the packaging.

The term "stable during the storage period" refers to the period during which the solution remains within the limits of potential chemical degradation products and the pH value assuming normal storage conditions of approximately 25 °C. Generally, the limit for assay are within +/-10 %, preferably +/-5 % as compared to the nominal filling concentration of the active pharmaceutical ingredient according to EMA guideline 3AQ11A. The limit for 2-methylaniline according to the specification of the Pharmacopoeia Europaea monography 1363 (Ph. Eur. 11. 4) is at a concentration of 0.1 % 2-methylaniline relative to the prilocaine concentration. The stated limits for 5-HMF are chosen according to USP monograph "Dextrose Injection" and BP monograph "Glucose Infusion", as 5-HMF is an impurity that arises due to glucose degradation. To further increase patient safety, the 5-HMF limits for a stable prilocaine solution comprising glucose are preferably even lower (as described in the according chapter). The limits for the pH value are chosen accordingly so that the degradation is minimized.

The term "stable" or "storage-stable" refers to the solution remaining within the limits of potential chemical degradation products and the pH assuming normal storage conditions of approximately 25 °C.

The term "pH at the beginning of the storage period" is used for the pH value at the end of the preparation process, after finalising step (d) or optionally step (e)..

Wherein the term "% (w/w)" is used it is referred to weight percentage in relation to the total amount of weight of the solution, if not explicitly stated otherwise.

The 5-HMF content in the solution is determined by HPLC measurement with an internal standard measurement.

The 2-methylaniline content in the solution is determined by HPLC measurement with an internal standard measurement.

### Examples

### Methods:

All standard and sample solutions used in HPLC related substance tests, given in the Pharmacopoeia Europaea (Ph. Eur. 11. 4) "Prilocaine HCl raw material" monograph, were used within 48 hours from preparation. The said solutions were stored under protection from light and at a temperature not exceeding 25 °C.

The HPLC experiments were processed by a HPLC Agilent 1100 or 1200 on a Nucleosil 100-5 C18 column (125 x 4.6 mm) with 0.45 mm filters and vacuum pump for mobile phase filtration.

The used solvents for the mobile phase were only used freshly prepared.

The mobile phase was prepared using phosphate buffer pH 8.0 (600 mL) and acetonitrile (400 mL), filtered and degassed under vacuum using a 0.45 µm filter. The phosphate buffer pH 8.0 was prepared by dissolving anhydrous Na₂HPO₄ (2.306 g) and NaH₂PO₄ dihydrate (0.2035 g) in 1000 ml of deionized water. The pH value was adjusted with NaOH (1 M) or H₃PO₄ (1 M), if necessary.

A prilocaine standard solution was prepared from prilocaine HCl (25.0 mg) dissolved in deionized water (50 mL), resulting in a corresponding solution of 0.5 mg/mL

A second standard solution containing the impurities 2-methylaniline and 5-hydoxymethylfurfural was prepared by dissolving prilocaine HCl (25 mg) in deionized water, adding 1 mL of a mother solution of 5-hydorxymethylfurfural and 0.1 mL of a mother solution of 2-methyaniline. The solution was brought to a volume of 50 mL with deionized water. The resulting solution corresponds to 0.5 mg/ml of prilocaine HCl, 0.5 µg/mL of 2-methylaniline and 5.0 µg/mL of 5-hydroxymethylfurfural.

The mother solution of 2-methyl-aniline was prepared by dissolving 2-methyl-aniline (25 mg) in 100 mL of deionized water.

The mother solution of 5-hydroxymethylfurfural was prepared by dissolving 2-methyl-aniline (25 mg) in 100 mL of deionized water.

A resolution solution was prepared by dissolving prilocaine HCl (3.0 mg) and impurity E (equivalent to Ph. Eur. definition) in 100 mL deionized water. The resulting solution was diluted tenfold with deionized water, corresponding to a solution of 3.0 µg/mL of prilocaine HCl and 3.0 µg/mL of Impurity E.

The HPLC experiments were conducted with a flow rate of 1.0 mL/min, a 100% isocratic mobile phase, an injection volume of 10 mL for assay and for impurities determination and a temperature of 25 °C.

The run time for a prilocaine HCl assay is 15 minutes. The run time for an impurity determination is 20 minutes.

The wavelength for the determination of prilocaine and related substances is set to 240 nm. The wavelength for the determination of 5-hydroxymethylfurfural is set to 284 nm.

### Example 1: Preparation of Prilocaine Hydrochloride 2 % hyperbaric solution

Glucose monohydrate (66.0 g) and prilocaine hydrochloride (20.0 g, corresponding to 17.16 g prilocaine free base) were dissolved in 900 mL of deionized water under constant stirring. The pH value was adjusted to the respective target pH with 0.1 M sodium hydroxide solution and, if needed, with 0.1 M hydrochloric acid. Subsequently, the solution was filled to a final volume of 1 L by addition of deionized water, filtered through a 0.2 µm filter, filled in plastic ampoules and heat sterilized at 121 °C with an F₀ value ≥ 15 min or at 112 °C with an F₀ value ≥ 8 min in an autoclave to obtain the final product.

The entire process was conducted without exclusion of oxygen under atmospheric conditions.

### Example 2: pH of prilocaine solution prior and after heat sterilization

Prilocaine solutions with different pH values (see Table 1) were prepared in accordance with Example 1. The pH value after sterilisation was determined and compared to the pH value prior to sterilisation (Table 1). The sterilisation was performed at 112 °C with an F₀ value ≥ 8 min.

Since the pH value of the solution can drop significantly during sterilisation and storage, it can be used as a quality parameter. It was shown in this example that the pH value drops during sterilisation. The extend to which the pH drops during heat sterilisation may depend on the pH value of the solution prior to sterilisation. Table 1 shows that the decrease in pH is least when the pH is at 6.5, 6.0 or 4.0 before sterilisation. According to the present example, the pH value prior to sterilization is advantageously selected to be at 6 or above.

**Table 1 pH value prior to and after heat sterilisation at 112 °C with an F₀ value ≥ 8 min.**

| **pH value prior to sterilisation** | **pH value after sterilisation** | **Decrease during heat sterilisation** |
|---|---|---|
| 6.5 | 6.2 | 0.3 |
| 6.0 | 5.7 | 0.3 |
| 5.5 | 5.0 | 0.5 |
| 5.2 | 4.5 | 0.7 |
| 5.0 | 4.2 | 0.8 |
| 4.5 | 4.0 | 0.5 |
| 4.0 | 3.8 | 0.2 |

### Example 3: pH development in the course of storage at different pH values prior to sterilisation

Solutions with different pH values were prepared in accordance with Example 1 (see Table 2).

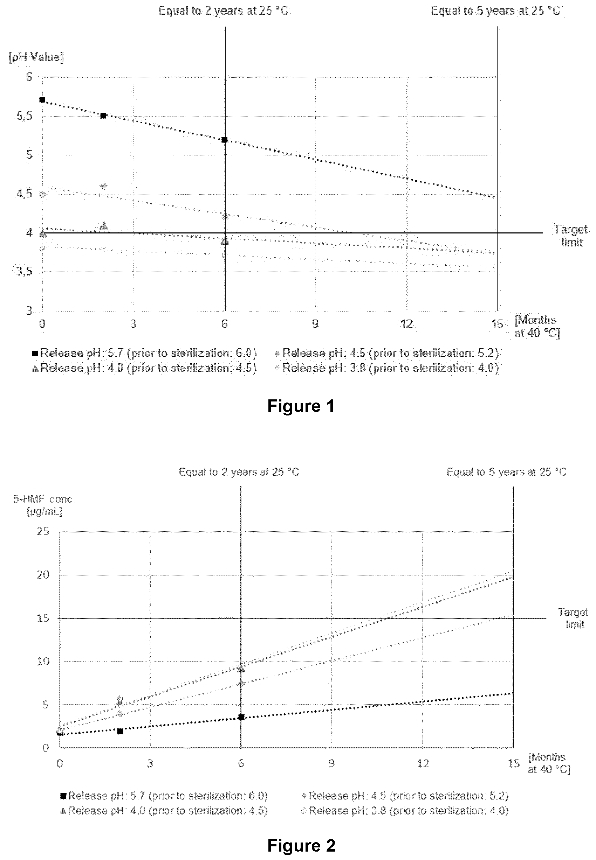

Resulting solutions of pH values between 4 and 6 were tested in accelerated storage conditions over 6 months at 40 °C. The solution with the highest pH value of 6 prior to sterilisation was well within the target limit (pH 4.0-6.0) with a value of 5.2 at the end of the study. The linear extrapolated data further show that the solution with a value of pH 6 prior to sterilisation remains within the target limit during 15 months of storage at 40 °C, corresponding to a five-year storage period at 25 °C (see Fig. 1). The sterilisation was performed at 112 °C with an F₀ value ≥ 8 min.

**Table 2 development of pH value during accelerated storage conditions at 40 °C**

| **pH value prior to sterilisation** | **pH value after sterilisation** | **pH value after 2 months** | **pH value after 6 months** | **Extrapolated pH at 15 months** |
|---|---|---|---|---|
| 6.0 | 5.7 | 5.5 | 5.2 | 4.45 |
| 5.2 | 4.5 | 4.6 | 4.2 | 3.75 |
| 4.5 | 4.0 | 4.1* | 3.9 | 3.75 |
| 4.0 | 3.8 | 3.8 | 3.7 | 3.55 |

| | | | | |
|---|---|---|---|---|
| *the value may appear increased due to rounding within the margin of error for pH electrodes that are around +/- 0.05 | | | | |

### Example 4: 5-HMF concentration in the course of storage at different pH

Solutions with different pH values were prepared in accordance with Example 1 (Table 3). The solutions were tested in accelerated storage conditions over 6 months at 40 °C and examined for the formation of the glucose impurity 5-hydroxymethylfurfural (5-HMF), determined by HPLC.

The pH value of the solution during storage also has a direct influence on the formation of a decomposition product of glucose, 5-HMF. A lower pH value favours the formation of 5-HMF and thus leads to an increase in the 5-HMF content in the solution during storage. Therefore, a higher pH value generates less 5-HMF during storage of the hyperbaric prilocaine solution. Table 3 shows a 5-HMF concentration of less than 4 µg/mL after six months of storage at 40 °C for a solution having a pH of 6.0 and above prior to sterilisation (see also Fig. 2, rhombus). The sterilisation was performed at 112 °C with an F₀ value ≥ 8 min.

**Table 3 5-HMF concentrations [µg/mL] in dependency of the pH value prior to sterilisation**

| **pH value prior to sterilisation** | **5-HMF before sterilisation** | **5-HMF after sterilisation** | **5-HMF after 2 months** | **5-HMF after 6 months** |
|---|---|---|---|---|
| 6.5 | 0.0 | 1.3 | 1.5 | N.A.* |
| 6.0 | 0.0 | 1.8 | 1.9 | 3.6 |
| 5.2 | 0.0 | 2.0 | 4.0 | 7.4 |
| 4.5 | 0.0 | 2.1 | 5.4 | 9.2 |
| 4.0 | 0.0 | 2.1 | 5.8 | 9.5 |

| | | | | |
|---|---|---|---|---|
| *N.A.: not applicable | | | | |

### Example 5: 2-methylaniline concentration at a pH of 5.7 after sterilisation

A solution with a pH value of 5.7 after sterilisation was prepared in accordance with Example 1. The sterilisation was performed at 112 °C with an F₀ value ≥ 8 min. The solution was tested in accelerated storage conditions over 6 months at 40 °C and examined for the formation of the prilocaine impurity 2-methylaniline, determined by HPLC.

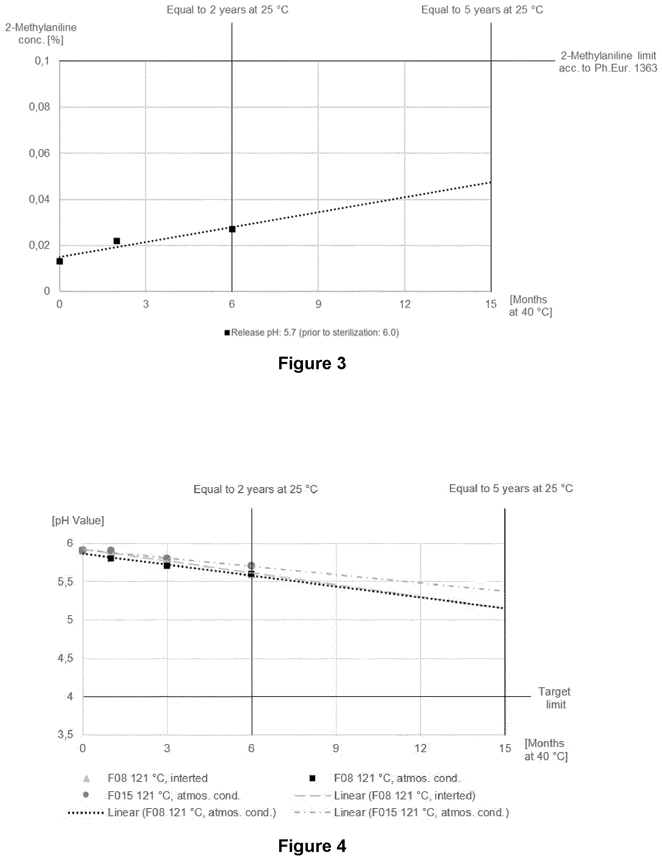

The main decomposition product of prilocaine is 2-methylaniline. The limit according to the specification of the Pharmacopoeia Europaea monography 1363 (Ph. Eur. 11. 4) is at a concentration of 0.1 %, relative to the prilocaine concentration. The 2-methylaniline content after six months of storage and 40 °C (corresponding to a 2-year storage period at 25 °C) for a solution with a pH value of 5.7 after sterilisation is well below the limit (see Fig. 3, Table 4). An extrapolation corresponding to a 5-year storage period at 25 °C (15 months storage at 40 °C) shows a final concentration of 2-methylaniline of 0.05 %

**Table 4 Relative 2-methylaniline concentration [%] at accelerated storage conditions**

| **After sterilisation** | **2 months** | **6 months** |
|---|---|---|
| 0.013 | 0.022 | 0.027 |

### Example 6: influence of oxygen on the pH value stability of the solution

A solution prepared according to Example 1 resulting in a pH of 5.9 after sterilization was exposed to environment (headspace) at different oxygen concentrations. Table 5 shows the data obtained from a 6-month stability test at 40 °C. In order to control the oxygen content, oxygen-impermeable glass vials were used as packaging material in these tests. The values obtained were extrapolated to a period of up to 15 months at 40 °C, corresponding to a storage period of 5 years at 25 °C.

The comparison of the different oxygen contents shows that there is no significant difference in the pH after 6 months for different oxygen concentrations in the headspace. Furthermore, 5-HMF, 2-methylaniline, and prilocaine assays were well within limits at all time points.

The solution further has a slightly higher pH when it is sterilized at 121 °C with an F₀ value ≥ 15 min (F₀15, Fig. 4 grey line), compared to a sterilisation at 112 °C with an F₀ value ≥ 8 min (F₀8). This is not surprising, as the thermal stress for the product is higher if it has to be sterilized for a longer time at a lower temperature compared to a shorter sterilization time at higher temperatures.

**Table 5 pH value behaviour under oxygen influence**

| **Oxygen content** | **Sterilisation conditions** | **pH after sterilization** | **pH after 1 month** | **pH after 3 months** | **pH after 6 months** |
|---|---|---|---|---|---|
| < 0.5 % | 112 °C / F₀8 | 5.9 | 5.9 | 5.8 | 5.6 |
| 21 % | 112 °C / F₀8 | 5.9 | 5.8 | 5.7 | 5.6 |
| 21 % | 121 °C / F₀15 | 5.9 | 5.9 | 5.8 | 5.7 |

### Example 7: Prilocaine assay after 6 months of storage at 40 °C

An attribute for assessing the stability of a prilocaine solution is the assay of prilocaine. To be considered stable during the storage period, the assay of prilocaine is within the acceptance criteria when it does not deviate by 10 %, preferably 5 % of the nominal value. In this example, 100 % of prilocaine content is related to 20 mg/ml.

A solution with a pH value of 5.7 after sterilisation was prepared in accordance with Example 1 and stored for six months at a temperature of 40 °C. The sterilisation was performed at 112 °C with an F₀ value ≥ 8 min. The solution was subsequently subjected to a quantification of the remaining prilocaine amount determined by means of HPLC.

The solution of prilocaine does not show any substantial degradation after storage of 6 months at 40 °C, which relates to 2 years of storage at room temperature and stays well within even the narrower 5 % limits.

### Embodiments

The present disclosure also pertains to the following numbered embodiments:
1. A pharmaceutical hyperbaric stable aqueous solution for injection, comprising prilocaine or a pharmaceutically acceptable salt thereof and glucose;
   wherein the solution has a 5-hydroxymethylfurfural concentration of less than 24 µg/mL over a storage period of at least five years; and
   wherein the solution has a pH ≥ 5.5 at the beginning of the storage period.
2. The solution according to embodiment 1, wherein the solution has a 5-hydroxymethylfurfural content of less than 20 µg/mL, preferably less than 15 µg/mL, further preferably 10 µg/mL, and still further preferably less than 7 µg/mL over the entire storage period; and/or
   wherein the solution has a 5-hydroxymethylfurfural content of less than 7 µg/mL, preferably less than 6 µg/mL, further preferably less than 5 µg/mL, and still further preferably less than 4 µg/mL after storage for 6 months at 40 °C, and/or
   wherein the solution has a 5-hydroxymethylfurfural content of less than 4 µg/mL, preferably less than 3 µg/mL, further preferably less than 2 µg/mL after storage for 2 months at 40 °C.
3. The solution according to any of the preceding embodiments, wherein the solution comprises prilocaine as free base or prilocaine HCl, preferably wherein the solution comprises prilocaine HCl.
4. The solution according to any of the preceding embodiments, wherein the amount of prilocaine or a pharmaceutically acceptable salt thereof is between 1.5 and 2.5 % (w/w), preferably between 1.8 and 2.2 % (w/w), more preferably between 1.9 and 2.1% (w/w) and most preferably at about 2 % (w/w), calculated on the basis of prilocaine hydrochloride.
5. The solution according to any of the preceding embodiments, wherein the amount of glucose is between 5 and 7 %(w/w), preferably between 5.4 and 6.6 %(w/w), more preferably between 5.6 and 6.4 %(w/w), even more preferably between 5.8 and 6.2 %(w/w) and most preferably at 6 % (w/w).
6. The solution according to any of the preceding embodiments, wherein the osmolarity of the solution is in the range of from 500 to 560 mOsm/kg, preferably in a range of from 520 to 550 mOsm/kg, and more preferably in a range of from 525 to 540 mOsm/kg.
7. The solution according to any of the preceding embodiments, wherein the pH value is ≥ 5.5, preferably ≥ 5.7, more preferably ≥ 5.9, even more preferably ≥ 6.0 or > 6.0, even more preferably ≥ 6.1, and most preferably ≥ 6.2, at the beginning of the storage period.
8. The solution according to any of the preceding embodiments, wherein solution is sterilized by heat sterilization at a temperature of at least 110 °C, preferably 121 °C; and/or wherein the sterilizing is performed with an F₀ value ≥ 8 minutes, preferably an F₀ value ≥ 15 minutes;
   or by filtration sterilization, preferably wherein the solution is sterilized by heat sterilization.
9. The solution according to any of the preceding embodiments for intrathecal injection, preferably for anaesthesia.
10. A packaging comprising a solution according to any of embodiments 1 to 8, wherein the packaging is oxygen permeable and/or allows for atmospheric conditions inside the packaging.
11. The packaging according to embodiment 10, wherein the packaging contains a headspace, and the headspace contains more than 2 % of oxygen, preferably more than 5 % of oxygen, more preferably more than 10 % of oxygen and most preferably more than 20 % of oxygen.
12. The packaging according to any of embodiments 10 and 11, wherein the packaging comprises at least one polymer, wherein the polymer is selected from the group consisting of a polyethylene, a polypropylene, a polyvinylchloride, a polystyrol, a polyamide, a polylactic acid, a polycarbonate, a polyacrylonitrile, a polyester, a cyclic olefin copolymer (COCs), a cyclic olefin polymer (COPs) and combinations thereof.
13. The packaging according to any of embodiments 10 to 12, wherein the packaging is sterilized by heat sterilization, at a temperature of at least 110 °C, preferably 121 °C; and/or wherein the sterilizing is performed with an F₀ value ≥ 8 minutes, preferably an F₀ value ≥ 15 minutes.
14. A method for preparing a solution according to any of embodiments 1 to 8, comprising the following steps in the order of:
   (a) Dissolving prilocaine or a pharmaceutically acceptable salt thereof and glucose in water for injection, thereby providing an aqueous solution;
   (b) Adjusting the pH value to ≥ 5.5 by addition of a suitable base or a suitable acid, thereby providing a pH-adjusted aqueous solution;
   (c) Optionally sterilizing the pH-adjusted aqueous solution by filtration;
   (d) Distributing the pH-adjusted aqueous solution into a packaging, resulting into a filled packaging; and
   (e) Optionally sterilizing the filled packaging.
15. A method according to embodiment 14, wherein at least one of the steps (a) to (e) is performed under atmospheric conditions or under inert conditions, preferably all of steps (a) to (e) are performed under atmospheric conditions or under inert conditions.
16. A method according to any of embodiments 14 and 15, wherein step (a) includes the addition of a suitable base or a suitable acid.
17. A method according to any of embodiments 14 to 16, wherein step (c) or step (e) is present.
18. The method according to any of the embodiments 14 to 17, wherein the base is selected from the group consisting of NaOH, KOH, Ca(OH)₂, Ca(OH)₂, NaHCO₃, Na₂CO₃, Na₃PO₄, CaCO₃, K₂CO₃, and KHCO₃.
19. The method according to any of the embodiments 14 to 18, wherein the acid is selected from the group consisting of HCl, citric acid, H₃PO₄, acetic acid and lactic acid.
20. The method according to any of the embodiments 14 to 19, wherein the pH is in a range of between 5.5 to 6.5 after step (d), or optionally step (e) if present.
21. The method according to any of the embodiments 14 to 20, wherein the sterilizing is performed at a temperature of at least 110 °C, preferably 121 °C; and/or
   wherein the sterilizing is performed with an F₀ value ≥ 8 minutes, preferably an F₀ value ≥ 15 minutes.

## Claims

1. A pharmaceutical hyperbaric stable aqueous solution for injection, comprising prilocaine or a pharmaceutically acceptable salt thereof and glucose;
wherein the solution has a 5-hydroxymethylfurfural concentration of less than 24 µg/mL over a storage period of at least five years; and
wherein the solution has a pH ≥ 5.5 at the beginning of the storage period.

2. The solution according to claim 1, wherein the solution has a 5-hydroxymethylfurfural content of less than 20 µg/mL, preferably less than 15 µg/mL, further preferably 10 µg/mL, and still further preferably less than 7 µg/mL over the entire storage period; and/or
wherein the solution has a 5-hydroxymethylfurfural content of less than 7 µg/mL, preferably less than 6 µg/mL, further preferably less than 5 µg/mL, and still further preferably less than 4 µg/mL after storage for 6 months at 40 °C, and/or
wherein the solution has a 5-hydroxymethylfurfural content of less than 4 µg/mL, preferably less than 3 µg/mL, further preferably less than 2 µg/mL after storage for 2 months at 40 °C.

3. The solution according to any of the preceding claims, wherein the solution comprises prilocaine as free base or prilocaine HCl, preferably wherein the solution comprises prilocaine HCl; and / or wherein the amount of prilocaine or a pharmaceutically acceptable salt thereof is between 1.5 and 2.5 % (w/w), preferably between 1.8 and 2.2 % (w/w), more preferably between 1.9 and 2.1% (w/w) and most preferably at about 2 % (w/w), calculated on the basis of prilocaine hydrochloride.

4. The solution according to any of the preceding claims, wherein the amount of glucose is between 5 and 7 %(w/w), preferably between 5.4 and 6.6 %(w/w), more preferably between 5.6 and 6.4 %(w/w), even more preferably between 5.8 and 6.2 %(w/w) and most preferably at 6 % (w/w), and / or wherein the osmolarity of the solution is in the range of from 500 to 560 mOsm/kg, preferably in a range of from 520 to 550 mOsm/kg, and more preferably in a range of from 525 to 540 mOsm/kg.

5. The solution according to any of the preceding claims, wherein the pH value is ≥ 5.5, preferably ≥ 5.7, more preferably ≥ 5.9, even more preferably ≥ 6.0 or > 6.0, even more preferably ≥ 6.1, and most preferably ≥ 6.2, at the beginning of the storage period.

6. The solution according to any of the preceding claims, wherein solution is sterilized by heat sterilization at a temperature of at least 110 °C, preferably 121 °C; and/or wherein the sterilizing is performed with an F₀ value ≥ 8 minutes, preferably an F₀ value ≥ 15 minutes;
or by filtration sterilization, preferably wherein the solution is sterilized by heat sterilization.

7. The solution according to any of the preceding claims for intrathecal injection, preferably for anaesthesia.

8. A packaging comprising a solution according to any of claims 1 to 6, wherein the packaging is oxygen permeable and/or allows for atmospheric conditions inside the packaging; and / or wherein the packaging contains a headspace, and the headspace contains more than 2 % of oxygen, preferably more than 5 % of oxygen, more preferably more than 10 % of oxygen and most preferably more than 20 % of oxygen; and / or wherein the packaging comprises at least one polymer, wherein the polymer is selected from the group consisting of a polyethylene, a polypropylene, a polyvinylchloride, a polystyrol, a polyamide, a polylactic acid, a polycarbonate, a polyacrylonitrile, a polyester, a cyclic olefin copolymer (COCs), a cyclic olefin polymer (COPs) and combinations thereof.

9. The packaging according to claim 8, wherein the packaging is sterilized by heat sterilization, at a temperature of at least 110 °C, preferably 121 °C; and/or wherein the sterilizing is performed with an F₀ value ≥ 8 minutes, preferably an F₀ value ≥ 15 minutes.

10. A method for preparing a solution according to any of claims 1 to 6, comprising the following steps in the order of:
(a) Dissolving prilocaine or a pharmaceutically acceptable salt thereof and glucose in water for injection, thereby providing an aqueous solution;
(b) Adjusting the pH value to ≥ 5.5 by addition of a suitable base or a suitable acid, thereby providing a pH-adjusted aqueous solution;
(c) Optionally sterilizing the pH-adjusted aqueous solution by filtration;
(d) Distributing the pH-adjusted aqueous solution into a packaging, resulting into a filled packaging; and
(e) Optionally sterilizing the filled packaging.

11. The method according to claim 10, wherein at least one of the steps (a) to (e) is performed under atmospheric conditions or under inert conditions, preferably all of steps (a) to (e) are performed under atmospheric conditions or under inert conditions.

12. The method according to any of claims 10 or 11, wherein step (a) includes the addition of a suitable base or a suitable acid, and / or wherein the base is selected from the group consisting of NaOH, KOH, Ca(OH)₂, Ca(OH)₂, NaHCO₃, Na₂CO₃, Na₃PO₄, CaCO₃, K₂CO₃, and KHCO₃, and / or wherein the acid is selected from the group consisting of HCl, citric acid, H₃PO₄, acetic acid and lactic acid.

13. The method according to any of claims 10 to 12, wherein step (c) or step (e) is present.

14. The method according to any of the claims 10 to 13, wherein the pH is in a range of between 5.5 to 6.5 after step (d), or optionally step (e) if present.

15. The method according to any of the claims 10 to 14, wherein the sterilizing is performed at a temperature of at least 110 °C, preferably 121 °C; and/or
wherein the sterilizing is performed with an F₀ value ≥ 8 minutes, preferably an F₀ value ≥ 15 minutes.
